# EUROPÄISCHE PATENTANMELDUNG

(11) **EP 3 261 005 A1**
(43) Veröffentlichungstag der Anmeldung: **27.12.2017**
(21) Anmeldenummer: 17177739.4
(22) Anmeldetag: 23.06.2017
(51) Int. Cl.: G06F 19/00, G06F 1/16, A61B 5/00, A61B 5/0404, H04M 1/04, G06F 1/20, A45C 11/00

(54) **TABLETEINHEIT ZUR ERMITTLUNG UND AUSWERTUNG VON PFLEGEDATEN UND/ODER VERSORGUNGSDATEN EINES MENSCHEN SOWIE VERFAHREN ZUR ÜBERPRÜFUNG EINER PFLEGE- UND/ODER VERSORGUNGSSITUATION EINES MENSCHEN**

(30) Priorität: 24.06.2016 DE 102016111652
(71) Anmelder: Riesinger, Michael, 48153 Münster (DE); Jansing, Frank, 48301 Nottuln (DE)
(72) Erfinder: Riesinger, Michael, 48153 Münster (DE); Jansing, Frank, 48301 Nottuln (DE)
(74) Vertreter: Michalski Hüttermann & Partner Patentanwälte mbB

(57) **Zusammenfassung**

Es ist eine Tableteinheit (10) zur Ermittlung und Auswertung von Pflegedaten und/oder Versorgungsdaten vorgesehen mit einer ein Display (18) aufweisenden Rechnereinheit (12), einem die Rechnereinheit (12) aufnehmenden Gehäuse (14), mindestens einer in einem zwischen dem Gehäuse (14) und der Rechnereinheit (12) ausgebildeten Hohlraum (36) angeordneten Messeinrichtung (34) zur Ermittlung von Pflegedaten und/oder Versorgungsdaten, wobei die Messeinrichtung (34) mit dem Gehäuse (14) befestigt ist und über eine Datenverbindung zur Übertragung der ermittelten Daten mit der Rechnereinheit (12) verbunden ist, und einem in der Rechnereinheit (12) als Computerprogrammprodukt gespeicherten Expertensystem zur Auswertung der ermittelten Daten, wobei die Rechnereinheit (12) ausgestaltet ist über das Display (18) mit einer Bedienperson zur Ermittlung der Daten in Kommunikation zu treten. Dadurch ist eine schnelle und sichere Überprüfung und/oder Beurteilung eines durch vorgegebene Kriterien definierten Pflegezustands ermöglicht.

## Beschreibung

Die Erfindung betrifft eine Tableteinheit und ein Verfahren, mit deren Hilfe Pflegedaten und/oder Versorgungsdaten von pflegebedürftigen Menschen ermittelt und ausgewertet werden können, um die Pflege- und Versorgungssituation dieses Menschen überprüfen und/oder beurteilen zu können.

Aus EP 2 439 670 A2 ist ein Schreibbrett mit einem elektronischen Display bekannt, das mit Hilfe von an dem Schreibbrett angebrachten Messgeräten Pflegedaten und/oder Versorgungsdaten für die Pflege- und/oder Versorgungssituation von Menschen ermitteln und mit Hilfe eines in einer Speichereinheit gespeicherten Expertensystems auswerten kann. Hierzu können auf dem Display abzuprüfende Daten abgefragt und die Messergebnisse der hierzu verwendeten Messgeräte eingegeben werden. Das Expertensystem kann die Messergebnisse mit Referenzdaten vergleichen und bei nicht befriedenden Antworten Verbesserungsvorschläge unterbreiten.

Insbesondere bei Institutionen, die Pflege und Hilfe für hilfs- und pflegedürftige Personen bereitstellen (Pflegeinstitutionen) besteht ein ständiges Bedürfnis einen durch vorgegebene Kriterien definierten Pflegezustand schnell, sicher und nachhaltig überprüfen und/oder beurteilen zu können.

Es ist die Aufgabe der Erfindung Maßnahmen anzugeben, die eine schnelle und sichere Überprüfung und/oder Beurteilung eines durch vorgegebene Kriterien definierten Pflegezustands ermöglichen.

Die Lösung der Aufgabe erfolgt erfindungsgemäß durch eine Tableteinheit mit den Merkmalen des Anspruchs 1 sowie ein Verfahren mit den Merkmalen des Anspruchs 8. Bevorzugte Ausgestaltungen der Erfindung sind in den Unteransprüchen angegeben, die jeweils einzeln oder in Kombination einen Aspekt der Erfindung darstellen können.

Erfindungsgemäß ist eine Tableteinheit zur Ermittlung und Auswertung von Pflegedaten und/oder Versorgungsdaten für die Pflege- und/oder Versorgungssituation von Menschen, insbesondere Menschen in geriatrischer und/oder palliativmedizinischer Behandlung vorgesehen mit einer ein Display aufweisenden, insbesondere als Tablet-Computer ausgestalteten, Rechnereinheit, einem die Rechnereinheit aufnehmenden Gehäuse, mindestens einer in einem zwischen dem Gehäuse und der Rechnereinheit ausgebildeten Hohlraum angeordneten Messeinrichtung zur Ermittlung von Pflegedaten und/oder Versorgungsdaten für die Pflege- und/oder Versorgungssituation von Menschen, insbesondere Menschen in geriatrischer und/oder palliativmedizinischer Behandlung, wobei die Messeinrichtung mit dem Gehäuse befestigt ist und über eine Datenverbindung zur Übertragung der ermittelten Daten mit der Rechnereinheit verbunden ist, und einem in der Rechnereinheit als Computerprogrammprodukt gespeicherten Expertensystem zur Auswertung der ermittelten Daten, wobei die Rechnereinheit ausgestaltet ist über das Display mit einer Bedienperson zur Ermittlung der Daten in Kommunikation zu treten.

Mit Hilfe der mindestens einen Messeinrichtung können bei einer Visite schnell und effizient allgemeine Messdaten, beispielsweise über das Raumklima im Bewohnerzimmer und/oder des Wohnumfelds mit hoher Genauigkeit ermittelt werden. Dadurch ist es nicht erforderlich, durch eine Befragung von Personen die Erfüllung von Kriterien für einen bestimmten Sollzustand, beispielsweise Kriterien über das Raumklima, erfragen zu müssen, wodurch Fehlerquellen vermieden sind. Zudem kann die jeweilige Messeinrichtung ihre Messergebnisse über die Datenleitung unmittelbar der Rechnereinheit zur Verfügung stellen. Dadurch können Ablesefehler beim Ablesen der Messeinrichtung und Eingabefehler beim Eingeben der Messdaten in die Rechnereinheit vermieden werden. Die Abprüfung derartiger Kriterien kann dadurch genauer und sicherer erfolgen. Unnötige Fehler und Ungenauigkeiten können vermieden werden. Ein Zusatzaufwand, während der Befragung mit Hilfe von erst zu beschaffenden Messgeräten Messungen durchzuführen, wird dadurch vermieden. Ferner kann die Befragung von Personen auf wenige Kriterien reduziert werden, wodurch die Überprüfung und Bewertung der Erreichung eines Sollzustands schneller und nachhaltiger erfolgen kann. Eine Störung der Arbeitsroutine durch die Befragung kann dadurch minimiert werden.

Zudem sind die Messeinrichtungen in dem zwischen dem Gehäuse und der Rechnereinheit ausgebildeten Hohlraum geschützt aufgenommen. Eine Beschädigung oder Verschmutzung der Messeinrichtungen kann dadurch vermieden werden. Der Hohlraum kann insbesondere genügend Freiraum bereitstellen, dass eine aktive und/oder passive Kühlung der Rechnereinheit und/oder der Messeinrichtungen ermöglicht ist. Eine Beeinträchtigung der Leistungsfähigkeit der Rechnereinheit und/oder der Messeinrichtungen sowie eine Beeinträchtigung der Messgenauigkeit der Messeinrichtungen durch Wärmeeffekte kann dadurch vermieden werden. Durch die zwischen der Rechnereinheit und dem Gehäuse geschützt aufgenommenen und über die Datenverbindung mit der Rechnereinheit kommunizierenden Messeinrichtungen ist eine einfache und sichere Messung von Messdaten und Übertragung der Messdaten erreicht, so dass eine schnelle und sichere Überprüfung und/oder Beurteilung eines durch vorgegebene Kriterien definierten Pflegezustands ermöglicht ist.

Die Rechnereinheit kann als ein Tablet-Computer ausgestaltet sein, der bereits ein Display zur Darstellung von Informationen aufweist. Zudem weist der Tablet-Computer hinreichende Speicherkapazitäten und eine hinreichende Rechenleistung auf, um das das Expertensystem zu speichern und auszuführen. Zudem ist genug Speicherplatz vorhanden, um die Messdaten und die Ergebnisse des Expertensystems zu speichern. Zudem weist der Tablet-Computer üblicherweise eine Datenschnittstelle, beispielsweise einen USB-Anschluss, auf, mit der die Datenverbindung der Rechnereinheit mit der mindestens einen in dem Hohlraum des Gehäuses vorgesehenen Messeinrichtung ausgebildet werden kann.

In der Rechnereinheit sind besonders bevorzugt von dem Expertensystem für die zu untersuchende Pflegeeinrichtung benötigte Daten, beispielsweise Bettenanzahl, Anzahl der Bewohnerzimmer einer geriatrischen Pflegeeinrichtung vorgehalten, so dass diese Daten nicht bei jedem Informationserfassungsprozess erneut erfasst werden müssen. Dies erleichtert und beschleunigt die Erfassung der mit der Tableteinheit erhältlichen Informationen, wodurch Stress und eine unnötig erhöhte Fehleranfälligkeit bei den Bedienpersonen vermieden wird. Es ist möglich, dass die Rechnereinheit eine Tastatur aufweist oder das Anschließen einer Tastatur ermöglicht, so dass über die Tastatur von einer Bedienperson Daten eingegeben werden können, um die von dem Expertensystem auf dem Display angezeigten Fragen beantworten zu können. Vorzugsweise ist das Display als Touchscreen ausgestaltet, der das Eingeben von Daten über einen berührungsempfindlichen Bereich des Displays ermöglicht.

Im medizinischen Bereich ist es eine anerkannte und übliche Praxis, mit Hilfe eines Klemmbretts oder einem Tablet-Computer bei einer Visite personenbezogene Daten, beispielsweise Patientendaten, bereitzuhalten und ergänzende Informationen zu notieren. Die erfindungsgemäße Tableteinheit kann dadurch entsprechend einfach in die übliche Arbeitsroutine, beispielsweise einer Pflegeinstitution integriert werden, indem aus dem medizinischen Bereich bekannte Arbeitsabläufe in den Pflegebereich, insbesondere in den geriatrischen Bereich, übertragen werden. Hierbei wird die Erkenntnis ausgenutzt, dass in einer geriatrischen Pflegeeinrichtung beispielsweise im Gegensatz zu Hygieneanforderungen in einem Operationssaal eines Krankenhauses zur Überprüfung einer Pflege- und/oder Versorgungssituation fachärztliches Spezialwissen nicht erforderlich ist und die Übertragung von Arbeitsweisen aus dem medizinischen Bereich in den geriatrischen Bereich überraschenderweise kostengünstig und effizient erfolgen kann. Die Tableteinheit kann dadurch insbesondere zu nicht-diagnostischen und/oder nicht-therapeutischen Zwecken eingesetzt werden. Insbesondere ist die mindestens eine Messeinrichtung auf den speziellen Verwendungszeck ausgestaltet im geriatrischen Bereich Informationen zu erfassen.
Dadurch ist es möglich die Messeinrichtung auf die zur Pflege- und/oder Versorgungssituation von Menschen in geriatrischer und/oder palliativmedizinischer Behandlung relevanten Daten zu beschränken, die sich beispielsweise aus einem vorgegebenen Kriterienkatalog ergeben. Dieser Kriterienkatalog kann beispielsweise durch ein Qualitätsmanagement und/oder gesetzliche Vorgaben vorgegeben werden. Beispielsweise kann der Sollzustand, insbesondere einzuhaltende Mindeststandards, für eine Pflegesituation und/oder eine Umgebungssituation für eine Pflegeeinrichtung durch Kriterien definiert werden, die freiwillig oder gesetzlich vorgegeben sein können, wobei freiwillig vorgegebene Qualitätskriterien insbesondere gesetzlich vorgegebene Kriterien übertreffen, um eine Pflege- und/oder Umgebungssituation nachhaltig zu ermöglichen, welche gesetzlich vorgesehene Mindeststandards übertreffen können. Insbesondere kann die Messeinrichtung dahingehend ausgestaltet sein, dass lediglich die Erfüllung beziehungsweise Nicht-Erfüllung eines bestimmten Kriteriums erfasst wird. Insbesondere wird durch die Messeinrichtung nicht eine zu messende Größe quantitativ ermittelt sondern lediglich qualitativ mit einem Soll-Wert verglichen. Dies vereinfacht den Aufbau der Messeinrichtung und erleichtert die Auswertung der erfassten Informationen, wodurch eine schnelle und sichere Überprüfung und/oder Beurteilung eines durch vorgegebene Kriterien definierten Sollzustands einer Pflege- und/oder Versorgungssituation von Menschen in geriatrischer und/oder palliativmedizinischer Behandlung ermöglicht ist.

Die Messeinrichtung ist insbesondere direkt mit dem Gehäuse befestigt. Insbesondere ist es mit Hilfe der mindestens einen Messeinrichtung möglich subjektiv zu bewertende weiche Kriterien durch objektiv mit Hilfe der Messeinrichtung überprüfbare harte Kriterien zu ersetzen, wodurch sich eine erhöhte Objektivität und Reliabilität ergibt. Darüber hinaus ist es möglich die Zeit zur Ermittlung der Daten zur Pflege- und/oder Versorgungssituation, insbesondere der Umgebungssituation, schnell und routiniert durchzuführen. Längeres Überlegen, ob ein bestimmtes Kriterium aus subjektiver Sicht erfüllt oder nicht erfüllt erscheint, kann vermieden werden. Dies führt dazu, dass in der Pflegeeinrichtung eingesetzte Personen für die Dokumentation der Pflege- und/oder Versorgungssituation entsprechend weniger Zeit benötigen und für diese Personen mehr bewohnernahe Zeit, beispielsweise für Gespräche und/oder Pflegeleistungen, verbleibt, wodurch das Wohlbefinden der Bewohner der Pflegeeinrichtung gesteigert werden kann.

Insbesondere ist als Messeinrichtung ein, insbesondere für Distanzen bis 50 cm vorgesehener, Kurzdistanzmesser und/oder ein insbesondere für Distanzen ab 50 cm vorgesehener, Langdistanzmesser und/oder ein Luftfeuchtigkeitsmessgerät und/oder ein Temperaturmessgerät und/oder ein Fotosensor zur Messung einer Lichtstärke und/oder ein Schallmessgerät zur Messung einer Lautstärke und/oder ein Beschleunigungssensor zur Messung einer Schwerkraftrichtung und/oder zur Messung einer Relativausrichtung der Tableteinheit zu einer Schwerkraftrichtung und/oder ein Infrarotsensor zur berührungslosen Messung einer Körpertemperatur und/oder eine Schwarzlichtlampe zur Abstrahlung von ultraviolettem Licht und/oder ein Farbmessgerät zur Detektion und/oder Unterscheidung verschiedener Farben und/oder ein Atmosphärenmesser, insbesondere zur Detektion der Luftzusammensetzung, vorgesehen. Erforderlichenfalls kann für die jeweilige Messeinrichtung in dem Gehäuse eine Öffnung vorgesehen sein, durch die hindurch ein Sensor der jeweiligen Messeinrichtung mit dem in der Umgebung befindlichen Messobjekt interagieren kann. Die Öffnung kann hierbei als Durchgangsöffnung ausgestaltet sein, so dass die Messeinrichtung unmittelbar der Umgebung ausgesetzt ist. Alternativ kann die Öffnung, beispielsweise bei einer auf einem optischen Prinzip basierenden Messung, durch einen im Wesentlichen transparenten Deckel verschlossen sein. Mit Hilfe der Messeinrichtungen können relevante Daten für die Dokumentation der Pflege- und/oder Versorgungssituation erfasst und von dem Expertensystem ausgewertet werden. Beispielsweise kann mit Hilfe des Beschleunigungssensors die Tableteinheit waagerecht ausgerichtet werden, um mit Hilfe des Kurzdistanzmessers zu überprüfen, ob in Bezug zu einer Matratze eines Bewohnerbetts das Bettgitter am seitlichen Rand des Bewohnerbetts eine erforderliche Mindesthöhe ("C-Maß") aufweist, damit der Bewohner des überprüften Bewohnerzimmers nicht versehentlich aus dem Bett fallen kann. Durch die Schwarzlichtlampe können auch ansonsten nicht gut sichtbar voneinander abgrenzbare Bereiche erkannt werden, die unter einer ansonsten intakten Oberfläche Beeinträchtigungen vermuten lassen. Durch das Farbmessgerät kann eine bestimmte Farbe identifiziert werden, wodurch es beispielsweise möglich ist eine Verfärbung als eine bestimmte Schimmelart erkennen zu können. Durch den Atmosphärenmesser kann beispielsweise ermittelt werden, ob eine zu einem guten Sauerstoffgehalt in der Atmosphäre führende ausreichende Lüftung eines Zimmers vorliegt.

Vorzugsweise weist die Rechnereinheit eine Kamera auf, wobei das Gehäuse eine Sichtöffnung für die Kamera aufweist. Insbesondere wenn die Rechnereinheit als Tablet-Computer ausgestaltet ist, ist häufig eine Kamera bereits eingebaut, so dass es nicht erforderlich ist mit dem Gehäuse eine weitere als Kamera ausgestaltete Messeinrichtung zu verbinden. Stattdessen kann es ausreichend sein in dem Gehäuse für die Kamera der Rechnereinheit die Sichtöffnung vorzusehen, die insbesondere als ein im Wesentlichen transparentes Fenster ausgestaltet ist.

Besonders bevorzugt weist die Rechnereinheit einen internen Datenanschluss zum Datenaustausch mit der mindestens einen Messeinrichtung auf, wobei mehrere Messeinrichtungen über den identischen Datenanschluss mit der Rechnereinheit verbunden sind. Dadurch ist nur es möglich nur genau einen Datenanschluss für den Anschluss der Messeinrichtungen an die Rechnereinheit vorzusehen. Ein weiterer Datenanschluss der Rechnereinheit kann dadurch für andere Zwecke freibleiben. Vorzugsweise sind sämtliche mit dem Gehäuse verbundenen Messeinrichtungen über den identischen gemeinsamen Datenanschluss an der Rechnereinheit angeschlossen. Beispielsweise können die Messeinrichtungen mit einer gemeinsamen mit dem Gehäuse verbundenen Zentraleinheit kommunizieren, in der die verschiedenen Signale der Messeinrichtungen aufgenommen und beispielsweise über ein vordefiniertes Datenprotokoll an den gemeinsamen mit der Zentraleinheit kommunizierenden Datenanschluss der Rechnereinheit in einer vordefinierten Form weitergeleitet werden. Obwohl in dem Hohlraum zwischen dem Gehäuse und der Rechnereinheit eine Vielzahl an Messeinrichtungen vorgesehen sein kann, ist ein einzelner in der Rechnereinheit vorgesehener Datenanschluss bereits ausreichend, um die Vielzahl an Messeinrichtungen auslesen zu können. Der Datenanschluss kann beispielsweise als USB-Anschluss ausgestaltet sein.

Insbesondere weist das Gehäuse und/oder die Rechnereinheit einen Datenanschluss zum Anschluss eines externen Messgeräts, insbesondere einen Biosscanner zur Erfassung von Vitalfunktionen eines Menschen, auf. Durch diesen Datenanschluss kann die Rechnereinheit auch mit einem Messgerät kommunizieren, das nicht in der Tableteinheit integriert ist. Beispielsweise kann mit Hilfe dieses Datenanschlusses eine Überwachungseinheit zur Erfassung von Vitaldaten eines Patienten angeschlossen werden, so dass die Tableteinheit zeitweise als ein Biomonitor genutzt werden kann. Die Vitaldaten, beispielsweise Puls, Atemfrequenz, Körpertemperatur etc., des Patienten können dadurch einfach erfasst und von dem gespeicherten Expertensystem ausgewertet werden, beispielsweise um zu überprüfen, ob das Raumklima für den Patienten angenehm eingestellt ist. So könnte beispielsweise bei einer erhöhten Körpertemperatur des Patienten eine leichte Absenkung der Raumtemperatur um 1 - 2 K empfohlen werden, um eine Raumtemperatur näher am unteren Rand eines zulässigen Raumtemperaturbereichs einzustellen.

Vorzugsweise weist das Gehäuse und/oder die Rechnereinheit einen Scanner zur optischen Erfassung eines maschinenlesbaren Codes, insbesondere Bar-Code und/oder QR-Code, auf, wobei insbesondere der Scanner zur dreidimensionalen Abtastung von Gebäudeteilen ausgestaltet ist. Der Scanner kann vorzugsweise durch eine Kamera ausgebildet sein, die insbesondere in der Rechnereinheit integriert ist. Mit Hilfe einer auf der Rechnereinheit ausgeführten Anwendung ("App") kann der maschinenlesbare Code erfasst und hinterlegten Daten zugeordnet werden. Beispielsweise kann einem Bewohnerzimmer ein bestimmter Code zugeordnet sein, so dass das Expertensystem abfragen kann, ob bestimmte Pflegehilfsmittel und/oder Pflegeunterstützungsgeräte vorhanden sind, die für dieses Bewohnerzimmer vorgesehen sind. Die Pflegehilfsmittel und/oder Pflegeunterstützungsgeräte können einen maschinenlesbaren Code aufweisen, die von dem Scanner, insbesondere automatisch, erfasst werden können, so dass das Vorhandensein der entsprechenden Hilfsmittel bestätigt werden kann. Zudem kann jedes Hilfsmittel einen eigenen maschinenlesbaren Code aufweisen, so dass aus dem eingelesenen Code bestimmte Wartungsaufgaben abgeleitet werden können. So kann beispielsweise das Alter und/oder eine Nutzungsdauer eines bestimmten Hilfsmittels ermittelt werden, sowie ob bestimmte Wartungsaufgaben überfällig oder demnächst fällig sind. Dies ermöglicht ein proaktives Versorgungsmanagement von Hilfsmitteln für die Pflege. Zudem kann beispielsweise ein an einer bestimmten Stelle nicht vorgesehenes Pflegeunterstützungsgerät gescannt werden, um herauszufinden an welcher Stelle dieses Pflegeunterstützungsgerät tatsächlich vorgesehen ist. Besonders bevorzugt können mit Hilfe des eingelesenen Codes bestimmte bauliche Anforderungen für die aktuelle Gebäuderegion abgefragt werden. So können beispielsweise die Anforderungen für einen Fluchtweg und/oder Brandschutzmaßnahmen erkannt werden. Mit Hilfe der dreidimensionalen Abtastung von Gebäudeteilen kann automatisch erkannt werden, ob ein designierter Fluchtweg verstellt ist, so dass von dem Expertensystem eine Empfehlung ausgegeben werden kann einen verstellten Fluchtweg an einer bestimmten Stelle freizugeben. Zudem kann mit Hilfe des Scanners eine Brandschutztür erkannt werden, so dass überprüft werden kann, ob diese Tür selbständig schließen kann.

Besonders bevorzugt weist das Gehäuse und/oder die Rechnereinheit ein Positionsbestimmungssystem zur dreidimensionalen Ortung, insbesondere zur Ermittlung eines Gebäudeteils, auf. Das Positionsbestimmungssystem kann beispielsweise auf einer satellitengestützen Positionsbestimmung beruhen. Es ist aber auch möglich, dass innerhalb oder in der Nähe des zu untersuchenden Gebäudeteils von dem Positionsbestimmungssystem erfassbare Referenzpunkte vorgesehen sind, aus denen das Positionsbestimmungssystem den aktuellen Ort in drei Koordinatenrichtungen ermitteln kann. In Kenntnis des genauen aktuellen Orts kann das Expertensystem für diesen Gebäudebereich relevante Fragen abprüfen lassen und gegebenenfalls nachhalten, welche Gebäudeteile noch inspiziert werden sollten. So kann beispielsweise überprüft werden, ob bestimmte Hilfsmittel, insbesondere Pflegehilfsmittel und/oder Pflegeunterstützungsgeräte, in einem bestimmten Gebäudeteil, bei dem es sich beispielsweise um ein Bewohnerzimmer eines Patienten mit bestimmten hinterlegten Pflegeanforderungen handelt, vorhanden und funktionsfähig sind. Zudem können auch Gebäudeteile außerhalb von Bewohnerzimmern und/oder im Außenbereich des zu untersuchenden Gebäudes in die Überprüfung mit Hilfe des Expertensystems einbezogen werden, beispielsweise um Brandschutzmaßnahmen und Fluchtwege zu identifizieren und zu überprüfen.

Insbesondere weist das Gehäuse und/oder die Rechnereinheit eine drahtlose Kommunikationseinheit zum Abrufen von Pflegerätedaten und/oder Pflegeortsdaten auf. Die für einen bestimmten Gebäudeteil vorgesehenen Geräte können in einer separaten Rechnereinheit, beispielsweise in einem Verwaltungsbereich, verwaltet werden, so dass auch in dieser separaten Rechnereinheit relevante Daten für die Pflegerätedaten nachgehalten und aktualisiert werden. Durch die drahtlose Kommunikationseinheit kann die Tableteinheit diese Daten abrufen und von dem Expertensystem überprüfen lassen, ob ein bestimmter Fragenkatalog für dieses Pflegegerät abgeprüft werden muss. Dadurch kann beispielsweise ein Wartungsbedarf ermittelt werden. Zudem kann die Tableteinheit mit Hilfe der drahtlosen Kommunikationseinheit abfragen, an welchem Ort das zu untersuchende Pflegegerät vorgesehen ist. Dadurch kann überprüft werden, ob das Pflegegerät am richtigen Ort ist oder an einem anderen Ort fehlt. Die hierzu erforderlichen teilweise recht umfangreichen Daten können außerhalb der Tableteinheit gespeichert sein und aktuell gehalten werden, so dass es nicht erforderlich ist diese Daten in der Rechnereinheit der Tableteinheit zu speichern.

Vorzugsweise weist das Gehäuse eine Belüftungsöffnung zur Kühlung der Rechnereinheit auf, wobei insbesondere in dem Hohlraum eine mit dem Gehäuse befestigte Lüftereinheit zur aktiven Kühlung der Rechnereinheit und/oder zur aktiven Kühlung zumindest einer Messeinrichtung vorgesehen ist. Die Belüftungsöffnung kann beispielweise mehrere Schlitze aufweisen, um einen Luftaustausch mit der Umgebung zu ermöglichen. Eine Kühleröffnung der Rechnereinheit ist dadurch nicht durch das Gehäuse verschlossen, sondern ermöglicht eine Kühlung über die in dem Gehäuse vorgesehene Belüftungsöffnung. Statt einer Beeinträchtigung der Kühlung der Rechnereinheit kann die Kühlung der Rechnereinheit durch die in dem Gehäuse vorgesehene Lüftereinheit durch eine zusätzliche aktive Kühlung sogar verbessert sein. Zudem können die in dem Hohlraum des Gehäuses vorgesehenen Messeinrichtungen gekühlt werden, so dass Messfehler durch Temperatureffekte vermieden werden können.

Besonders bevorzugt ist mit dem Gehäuse ein Tragehenkel zum Tragen und/oder Aufstellen der Tableteinheit schwenkbar und/oder verschiebbar befestigt ist, wobei insbesondere der Tragehenkel in einer versenkten Stellung oberflächenbündig in den Formverlauf des Gehäuses integriert ist. Der Tragehenkel kann den Transport der Tableteinheit erleichtern. Wenn der Tragehenkel nicht gebraucht wird, weil beispielsweise die Tableteinheit im Stehen wie ein Klemmbrett in der Hand gehalten wird, kann der Tragehenkel in dem Gehäuse versenkt werden, so dass der Tragehenkel nicht seitlich übersteht. Vorzugsweise kann der Tragehenkel in der versenkten Stellung einen Datenanschluss abdecken, der dadurch vor Umwelteinflüssen besser geschützt ist. Der Tragehenkel kann insbesondere in eine Position verschwenkt werden, in der die Tableteinheit auf einem Untergrund aufgestellt werden kann. Dies ermöglicht es, die Tableteinheit leicht abzustellen, um eine Überprüfung für den abzuarbeitenden Fragenkatalog des Expertensystems durchzuführen. Im aufgestellten Zustand kann eine Bedienperson leicht nachsehen, welche Überprüfungen durchgeführt werden sollen, auch wenn die Bedienperson die Tableteinheit nicht in der Hand hält oder aufgrund der aktuell durchgeführten Tätigkeit nicht in der Hand halten kann. In dem Gehäuse können für das Erreichen der einzelnen Stellungen des Tragehenkels Führungen und/oder lösbare Verrastungen vorgesehen sein.

Insbesondere ist mit dem Gehäuse eine Abdeckblende zur verliersicheren Aufnahme der Rechnereinheit zwischen dem Gehäuse und der Abdeckblende lösbar befestigt. Die Abdeckblende kann einen Teil der von dem Gehäuse weg weisenden Vorderseite der Rechnereinheit abdecken. Zudem kann das Gehäuse und/oder die Abdeckblende eine seitliche Bewegung der Rechnereinheit relativ zu dem Gehäuse blockieren. Wenn die Abdeckblende mit dem Gehäuse verbunden ist, kann die Rechnereinheit sicher aufgenommen sein. Hierbei kann die Abdeckblende insbesondere das Display der Rechnereinheit freilassen, insbesondere wenn das Display eine berührungsempfindliche Oberfläche zur Ausbildung eines Touchscreens aufweist. Durch die lösbare Befestigung der Abdeckblende mit dem Gehäuse kann die Rechnereinheit, beispielsweise bei einem Defekt leicht durch eine andere Rechnereinheit ausgetauscht werden. Es ist auch möglich die Rechnereinheit durch eine andere Rechnereinheit mit anderen Abmessungen auszutauschen. In diesem Fall kann eine auf die anderen Abmessungen der neuen Rechnereinheit angepasste Abdeckblende verwendet werden, die eine seitliche Relativbewegung der Rechnereinheit blockieren kann und im mit dem Gehäuse befestigten Zustand die neue Rechnereinheit zwischen dem Gehäuse und der Abdeckblende verklemmen kann. Ein Austausch des Gehäuses und der in dem Gehäuse vorgesehenen Messtechnik der mindestens einen Messeinrichtung ist dadurch nicht erforderlich.

Vorzugsweise ist eine Längenmesseinrichtung, insbesondere ein Lineal, in dem Gehäuse und/oder in der Abdeckblende, insbesondere durch Gravieren, vorgesehen. Dies erleichtert eine schnelle Längenmessung, indem die Tableteinheit lediglich an einer zu messenden Strecke gehalten werden kann. Dies bietet sich insbesondere an, wenn am Ende einer zu messenden Strecke ein Gegenfläche fehl, deren Abstand zu einer Seite der Tableteinheit mit Hilfe eines berührungslos messenden Distanzmessers gemessen werden kann.

Besonders bevorzugt weist das Gehäuse an einer zum Gehäuse weisenden Rückseite der Rechnereinheit anliegende Stützrippen auf, wobei zwischen dem Gehäuse, der Rückseite der Rechnereinheit und den Stützrippen Strömungskanäle zur aktiven und/oder passiven Kühlung der Rechnereinheit ausgebildet sind. Die Stützrippen können an der Rechnereinheit angreifende Kräfte, beispielsweise wenn von Hand Daten eingegeben werden, an das Gehäuse abstützen. Gleichzeitig können die Stützrippen eine Luftströmung im Hohlraum begrenzen und einen bestimmten Strömungsweg vorgeben. Dies kann die aktive und/oder passive Kühlung der Rechnereinheit und/oder Messeinrichtungen verbessern. Gegebenenfalls können die Stützrippen aus einem metallischen Material hergestellt sein, so dass die Stützrippen in der Art von Kühlrippen durch Wärmeleitung Wärme von der Rechnereinheit aufnehmen und durch konvektiven Wärmeübergang zur Wärmeabfuhr abgeben können.

Insbesondere ist das Display für eine Bedienung mit einem Finger und/oder einem elektrischen Stift berührungsempfindlich ausgestaltet. Das Display kann insbesondere als Touchscreen ausgestaltet sein. Eine separate Tastatur kann dadurch eingespart werden.

Vorzugsweise ist ein mit der Rechnereinheit verbundenes Mikrophon zur Aufzeichnung von Sprache verbunden, wobei insbesondere die Rechnereinheit eine Spracherkennungssoftware zur Konvertierung der aufgezeichneten Sprache in elektronisch verarbeitbare Antworten auf Eingabeaufforderungen des Expertensystems aufweist. Dies ermöglicht es die Tableteinheit in der Art eines Diktiergeräts zu benutzen und bestimmte Anmerkungen in Sprachform und/oder mit Hilfe einer Spracherkennungssoftware in schriftlicher Form aufzunehmen. Zudem können von dem Expertensystem gestellte Fragen, für die eine Eingabe durch die Bedienperson erforderlich ist, auch mündlich beantwortet werden. Dies kann den Zeitaufwand zur Ermittlung der Daten zur Pflege- und/oder Versorgungssituation, insbesondere der Umgebungssituation, schnell und routiniert durchzuführen.

Besonders bevorzugt ist mit dem Gehäuse eine Klemme zum Festklemmen von Papier auf dem Display befestigt. Die Tableteinheit kann dadurch in der Art eines Klemmbretts verwendet werden. Dies ermöglich es Notizen mit der Tableteinheit zu befestigen. Beispielweise können Ausdrucke einer Herz-/Lungenmaschine mit Hilfe der Klemme mit der Tableteinheit verklemmt werden, um zur Umsetzung bestimmter Empfehlungen des Expertensystems relevante Daten zur Verfügung zu haben. Dies bietet sich insbesondere bei sensiblen Patientendaten an, die aus Datenschutzgründen nicht unnötig gespeichert werden sollen. Dennoch kann ein Entscheidungsträger, beispielsweise ein behandelnder Arzt, die Empfehlungen des Expertensystems , die hierzu relevanten Messdaten und den aktuellen Zustand eines Patienten in einen Kontext setzen, um die Pflegesituation des Patienten zu optimieren.

Die Erfindung betrifft ferner ein Verfahren zur Überprüfung und/oder Beurteilung eines durch vorgegebene Kriterien definierten Pflege- und/oder Versorgungssituation eines Menschen, insbesondere eines Menschen in geriatrischer und/oder palliativmedizinischer Behandlung, bei dem in einer Tableteinheit, die wie vorstehend beschrieben aus- und weitergebildet sein kann, auf Basis der vorgegebenen Kriterien Fragen zum Istzustand ermittelt werden, Antworten auf die Fragen detektiert und weiterverarbeitet werden, wobei bei der Weiterverarbeitung der Antworten durch einen Vergleich mit Referenzdaten die Antworten bewertet und nicht befriedigende Antworten identifiziert werden, und im Falle einer nicht befriedigenden Antwort mit Hilfe des gespeicherten Expertensystems Verbesserungsvorschläge unterbreitet werden, wobei die unterbreiteten Verbesserungsvorschläge insbesondere weitere Fragen aufweisen und auf Grundlage der weiteren Antworten auf die weiteren Fragen mit Hilfe des gespeicherten Expertensystems weitere Verbesserungsvorschläge unterbreitet werden. Durch die zwischen der Rechnereinheit und dem Gehäuse geschützt aufgenommenen und über die Datenverbindung mit der Rechnereinheit kommunizierenden Messeinrichtungen ist eine einfache und sichere Messung von Messdaten und Übertragung der Messdaten erreicht, so dass eine schnelle und sichere Überprüfung und/oder Beurteilung eines durch vorgegebene Kriterien definierten Pflegezustands ermöglicht ist.

Insbesondere wird bei einer mit Hilfe des mindestens einer Messeinrichtung der Tableteinheit zu beantwortenden Frage eine auf dem Messergebnis der Messeinrichtung basierende Antwort für die Frage automatisch vorgegeben und/oder automatisch beantwortet. Beispielsweise benötigt die Tableteinheit zur Überprüfung des Raumklimas eines Bewohnerzimmers eine Temperatur, die automatisch von einer als Thermometer ausgestalteten Messeinrichtung übernommen werden kann. Hierzu kann die Tableteinheit vorzugsweise mit Hilfe eines Positionsbestimmungssystems und einer hinterlegten Karte vorher überprüfen, ob sich die Tableteinheit überhaupt in einem Bewohnerzimmer befindet und/oder um welches Bewohnerzimmer mit welchen Vorgaben für das Raumklima es sich handelt. Gegebenenfalls kann vorgesehen sein, dass eine Bedienperson die automatisch ermittelten Antworten zumindest noch selber bestätigen muss. Dadurch können offensichtliche Messfehler vermieden werden und die Bedienperson erhält einen Überblick über die abgefragten Aspekte.

Vorzugsweise wird in Abhängigkeit von einem detektierten Pflegegerät und/oder einem detektierten Pflegeort ein dieses Pflegegerät und/oder ein diesen Pflegeort betreffender Fragenkatalog abgefragt. Dies ermöglicht es unterschiedliche Anforderungsprofile für eine individuelle Pflegesituation zu hinterlegen und abzuprüfen. Insbesondere können für unterschiedliche Pflegegerät verschiedene zu überprüfende Kriterien relevant werden, die auch vom jeweiligen Zeitpunkt der Überprüfung abhängig sein können. So kann beispielweise eine aufwendige Überprüfung eines bestimmten Pflegegeräts entfallen, wenn eine kürzlich erfolge Überprüfung stattgefunden hat und zu einem positiven Ergebnis geführt hat. Dies ermöglicht es bei der Überprüfung der Pflegesituation unnötige Überprüfungen zu vermeiden, wodurch die Überprüfung besonders schnell erfolgen kann.

Nachfolgend wird die Erfindung unter Bezugnahme auf die anliegenden Zeichnungen anhand eines bevorzugten Ausführungsbeispiels exemplarisch erläutert, wobei die nachfolgend dargestellten Merkmale sowohl jeweils einzeln als auch in Kombination einen Aspekt der Erfindung darstellen können. Es zeigen:
Fig. 1: eine schematische perspektivische Vorderansicht einer Tableteinheit in einer Tragstellung
Fig. 2: eine schematische perspektivische Rückansicht der Tableteinheit aus Fig. 1 in einer aufgestellten Stellung,
Fig. 3: eine schematische Seitenansicht der Tableteinheit aus Fig. 1,
Fig. 4: eine schematische Seitenansicht der Tableteinheit aus Fig. 2,
Fig.5: eine schematische Seitenansicht der Tableteinheit aus Fig. 1 in einer versenkten Stellung und
Fig. 6: eine schematische perspektivische teilweise durchsichtige Ansicht eines Gehäuses der Tableteinheit aus Fig. 1.

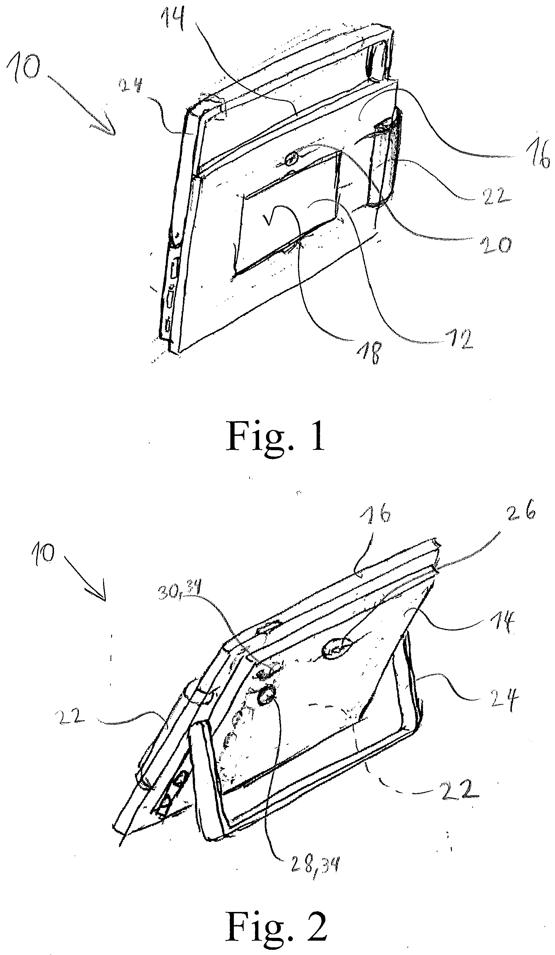

Die in Fig. 1 dargestellte Tableteinheit 10 weist eine als Tablet-Computer ausgestaltete Rechnereinheit 12 auf, die zwischen einem Gehäuse 14 und einer lösbar mit dem Gehäuse 14 verbundenen Abdeckblende 16 verliersicher und bewegungsfest aufgenommen ist. Die Abdeckblende 16 kann ein als Touchscreen ausgebildetes Display 18 aufweisen, auf dem ein in der Rechnereinheit 12 gespeichertes Expertensystem zu beantwortende Fragen ausgeben kann, um Pflegedaten und/oder Versorgungsdaten für die Pflege- und/oder Versorgungssituation von Menschen, insbesondere Menschen in geriatrischer und/oder palliativmedizinischer Behandlung, erfassen zu können. Um die Fragen auch mündlich beantworten zu können, kann ein Mikrofon 20 vorgesehen sein, das insbesondere mit einer Spracherkennungssoftware der Rechnereinheit 12 kommunizieren kann. Zudem ist mit dem Gehäuse 14 und/oder der Abdeckblende 16 eine Klemme 22 verbunden, mit deren Hilfe Schriftstücke festgeklemmt werden können. Die Tableteinheit 10 kann dadurch in der Art eines Klemmbretts verwendet werden.

Mit dem Gehäuse 14 ist zudem ein Tragehenkel 24 verschiedbar und schwenkbar verbunden. In der in Fig. 1 dargestellten ausgeschobenen Stellung kann die Tableteinheit 10 leicht am Tragehenkel 24 mitgenommen werden. In der in Fig. 2 dargestellten Stellung des Tragehenkels 24 kann die Tableteinheit 10 auf einem Untergrund aufgestellt werden. Die Tableteinheit 10 kann eine Kamera 26 aufweisen, die insbesondere von der Rechnereinheit 12 ausgebildet ist und durch eine in dem Gehäuse 14 ausgebildete Sichtöffnung hindurch Aufnahmen tätigen kann. Beispielsweise kann die Kamera maschinenlesbare Codes erfassen und/oder gegebenenfalls unterstützend zu einem separaten Scanner einen Raum dreidimensional abtasten. Zudem kann in dem Gehäuse 14 ein, insbesondere laserbasierter, Langdistanzmesser 28 und ein Kurzdistanzmesser 30 vorgesehen sein, die durch das Gehäuse 14 hindurch eine Distanz messen können. Hierzu können in dem Gehäuse 14 geeignete Öffnungen vorgesehen sein, um die Messung nicht zu beeinträchtigen.

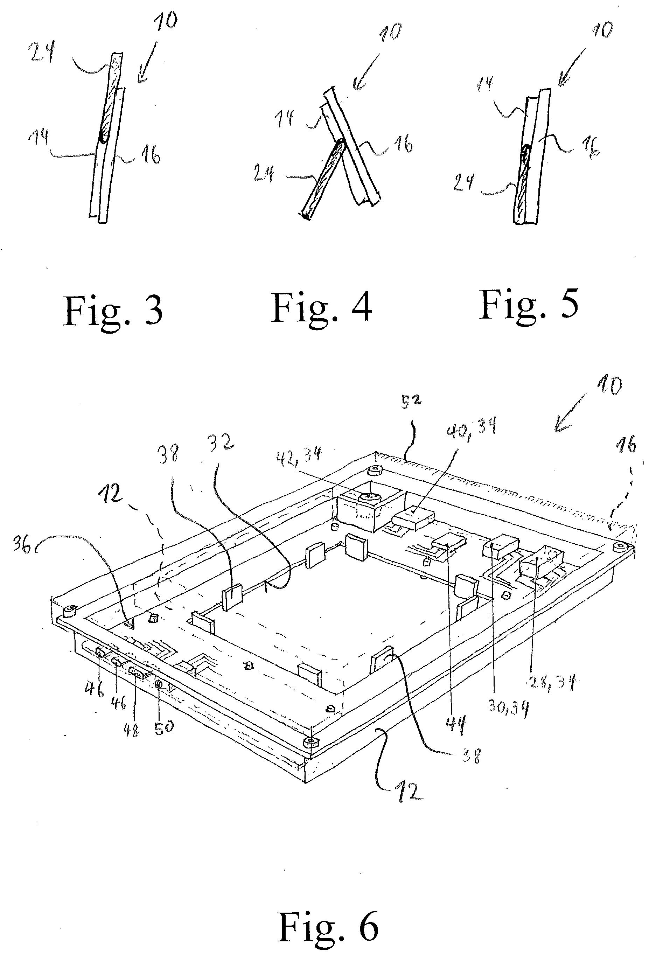

Wie in Fig. 3 dargestellt, kann der Tragehenkel 24 in der in Fig. 1 dargestellten ausgeschobenen Tragstellung leicht gegriffen werden, um die Tableteinheit 10 zu tragen. Die Tableteinheit weist hierbei eine geringe Dicke auf. Wie in Fig. 4 dargestellt, kann die Tableteinheit 10 in der in Fig. 2 dargestellten aufgestellten Stellung leicht auf einem Untergrund stabil aufgesetzt sein. Hierzu kann im Vergleich zu der in Fig. 1 und Fig. 3 dargestellten Tragstellung der Tragehenkel 24 um mehr als 180° verschwenkt sein und in der aufgestellten Stellung an einem Anschlag anliegen und/oder verrastet sein. In der in Fig. 5 dargestellten versenkten Stellung des Tragehenkels 24 liegt der Tragehenkel 24 oberflächenbündig an einer Rückseite des Gehäuses 14 und an einer umlaufenden Seitenwand der Abdeckblende 16 an. Die Abdeckblende 16 und das Gehäuse 14 bilden einen Stufe aus, in die der Traghenkel 24 versenkt aufgenommen und insbesondere verrastet sein kann. Die Tableteinheit 10 weist in der versenkten Stellung eine besonders geringe Erstreckung auf.

Wie in Fig. 6 dargestellt weist das Gehäuse 14 eine Belüftungsöffnung 32 auf, über welche die Rechnereinheit 12 und Messeinrichtungen 34 gekühlt werden können. Zwischen dem Gehäuse 14 und der Rechnereinheit 12 sowie der Abdeckblende 16 ist ein Hohlraum 36 ausgebildet, in dem die mit dem Gehäuse 14 fest verbundenen Messeinrichtungen 34 vorgesehen sind. Das Gehäuse 14 weist in dem Hohlraum 36 vorgesehene Stützrippen 38 auf, auf denen die Rechnereinheit 12 abgesetzt ist. Gleichzeitig können die Stützrippen 38 Strömungskanäle für eine aktive und/oder passive Kühlung der Rechnereinheit 12 und/oder der Messeinrichtungen 34 begrenzen. Als Messeinrichtungen 34 kann die Tableteinheit 10 neben dem Langdistanzmesser 28 und dem Kurzdistanzmesser 30 ein Temperaturmessgerät 40, ein Luftfeuchtigkeitsmessgerät 42 und weitere Messeinrichtungen 34 aufweisen. Zudem kann einen Datenanschluss 44 zum Anschluss eines externen Messgeräts, beispielweise zum Messen von Vitalfunktionen eines Patienten, aufweisen. Die in dem Hohlraum 36 vorgesehenen Messeinrichtungen 34 und über den Datenanschluss 44 angeschlossenen externen Messgeräte können über genau einen internen Datenanschluss mit der Rechnereinheit 12 kommunizieren. Zusätzlich kann die Tableteinheit 10 als weitere Datenanschlüsse einen oder mehr USB-Anschlüsse 46, einen Videoanschluss 48 und einen Ladeanschluss 50 zum Laden einer wiederaufladbaren Batterie der Tableteinheit 10 zur elektrischen Versorgung der Rechnereinheit 12. Es ist auch möglich ohne elektrisch betriebene Messeinrichtungen 34 eine Messung durchzuführen, beispielsweise indem in der Abdeckblende 16 ein Lineal 52 eingraviert ist, mit dem Längenmessungen vorgenommen werden können.

## Patentansprüche

1. Tableteinheit zur Ermittlung und Auswertung von Pflegedaten und/oder Versorgungsdaten für die Pflege- und/oder Versorgungssituation von Menschen, insbesondere Menschen in geriatrischer und/oder palliativmedizinischer Behandlung, mit
einer ein Display (18) aufweisenden, insbesondere als Tablet-Computer ausgestalteten, Rechnereinheit (12),
einem die Rechnereinheit (12) aufnehmenden Gehäuse (14),
mindestens einer in einem zwischen dem Gehäuse (14) und der Rechnereinheit (12) ausgebildeten Hohlraum (36) angeordneten Messeinrichtung (34) zur Ermittlung von Pflegedaten und/oder Versorgungsdaten für die Pflege- und/oder Versorgungssituation von Menschen, insbesondere Menschen in geriatrischer und/oder palliativmedizinischer Behandlung,
wobei die Messeinrichtung (34) mit dem Gehäuse (14) befestigt ist und über eine Datenverbindung zur Übertragung der ermittelten Daten mit der Rechnereinheit (12) verbunden ist, und
einem in der Rechnereinheit (12) als Computerprogrammprodukt gespeicherten Expertensystem zur Auswertung der ermittelten Daten, wobei die Rechnereinheit (12) ausgestaltet ist über das Display (18) mit einer Bedienperson zur Ermittlung der Daten in Kommunikation zu treten.

2. Tableteinheit nach Anspruch 1 **dadurch gekennzeichnet, dass** als Messeinrichtung (34) ein, insbesondere für Distanzen bis 50 cm vorgesehener, Kurzdistanzmesser (30) und/oder ein insbesondere für Distanzen ab 50 cm vorgesehener, Langdistanzmesser (28) und/oder ein Luftfeuchtigkeitsmessgerät (42) und/oder ein Temperaturmessgerät (40) und/oder ein Fotosensor zur Messung einer Lichtstärke und/oder ein Schallmessgerät zur Messung einer Lautstärke und/oder ein Beschleunigungssensor zur Messung einer Schwerkraftrichtung und/oder zur Messung einer Relativausrichtung der Tableteinheit zu einer Schwerkraftrichtung und/oder ein Infrarotsensor zur berührungslosen Messung einer Körpertemperatur vorgesehen ist.

3. Tableteinheit nach Anspruch 1 oder 2 **dadurch gekennzeichnet, dass** die Rechnereinheit (12) einen internen Datenanschluss zum Datenaustausch mit der mindestens einen Messeinrichtung (34) aufweist, wobei mehrere Messeinrichtungen (34) über den identischen Datenanschluss mit der Rechnereinheit (12) verbunden sind.

4. Tableteinheit nach einem der Ansprüche 1 bis 3 **dadurch gekennzeichnet, dass** das Gehäuse (14) und/oder die Rechnereinheit (12) einen Scanner zur optischen Erfassung eines maschinenlesbaren Codes, insbesondere Bar-Code und/oder QR-Code, aufweist, wobei insbesondere der Scanner zur dreidimensionalen Abtastung von Gebäudeteilen ausgestaltet ist.

5. Tableteinheit nach einem der Ansprüche 1 bis 4 **dadurch gekennzeichnet, dass** mit dem Gehäuse (14) ein Tragehenkel (24) zum Tragen und/oder Aufstellen der Tableteinheit (10) schwenkbar und/oder verschiebbar befestigt ist, wobei insbesondere der Tragehenkel (24) in einer versenkten Stellung oberflächenbündig in den Formverlauf des Gehäuses (14) integriert ist.

6. Tableteinheit nach einem der Ansprüche 1 bis 5 **dadurch gekennzeichnet, dass** mit dem Gehäuse (14) eine Abdeckblende (16) zur verliersicheren Aufnahme der Rechnereinheit (12) zwischen dem Gehäuse(14) und der Abdeckblende (16) lösbar befestigt ist.

7. Tableteinheit nach einem der Ansprüche 1 bis 6 **dadurch gekennzeichnet, dass** das Gehäuse (14) an einer zum Gehäuse (14) weisenden Rückseite der Rechnereinheit (12) anliegende Stützrippen (38) aufweist, wobei zwischen dem Gehäuse (14), der Rückseite der Rechnereinheit (12) und den Stützrippen (38) Strömungskanäle zur aktiven und/oder passiven Kühlung der Rechnereinheit (12) ausgebildet sind.

8. Verfahren zur Überprüfung und/oder Beurteilung eines durch vorgegebene Kriterien definierten Pflege- und/oder Versorgungssituation eines Menschen, insbesondere eines Menschen in geriatrischer und/oder palliativmedizinischer Behandlung, bei dem in einer Tableteinheit (10) nach einem der Ansprüche 1 bis 7 auf Basis der vorgegebenen Kriterien Fragen zum Istzustand ermittelt werden,
Antworten auf die Fragen detektiert und weiterverarbeitet werden, wobei bei der Weiterverarbeitung der Antworten durch einen Vergleich mit Referenzdaten die Antworten bewertet und nicht befriedigende Antworten identifiziert werden, und im Falle einer nicht befriedigenden Antwort mit Hilfe des gespeicherten Expertensystems Verbesserungsvorschläge unterbreitet werden, wobei die unterbreiteten Verbesserungsvorschläge insbesondere weitere Fragen aufweisen und auf Grundlage der weiteren Antworten auf die weiteren Fragen mit Hilfe des gespeicherten Expertensystems weitere Verbesserungsvorschläge unterbreitet werden.

9. Verfahren nach Anspruch 8, bei dem bei einer mit Hilfe der mindestens einen Messeinrichtung (34) der Tableteinheit (10) zu beantwortenden Frage eine auf dem Messergebnis der Messeinrichtung basierende Antwort für die Frage automatisch vorgegeben und/oder automatisch beantwortet wird.

10. Verfahren nach Anspruch 8 oder 9, bei dem in Abhängigkeit von einem detektierten Pflegegerät und/oder einem detektierten Pflegeort ein dieses Pflegegerät und/oder ein diesen Pflegeort betreffender Fragenkatalog abgefragt wird.
